**Europäisches Patentamt**

**European Patent Office**

**Office européen des brevets**

(11) Veröffentlichungsnummer: **0 100 050**
**A2**

(12) **EUROPÄISCHE PATENTANMELDUNG**

(21) Anmeldenummer: **83107028.9**

(22) Anmeldetag: **18.07.83**

(51) Int. Cl.³: **A 61 H 39/00**
**// A61N1/42**

(30) Priorität: **23.07.82 DE 3227505**
**23.07.82 DE 3227506**
**16.09.82 DE 3234339**

(43) Veröffentlichungstag der Anmeldung: **08.02.84**
**Patentblatt 84/6**

(84) Benannte Vertragsstaaten: **AT BE CH DE FR GB IT LI LU
NL SE**

(71) Anmelder: **Blum, Klaus-Jürgen, Dr., Kleinenbroicher
Strasse 65, D-4050 Mönchengladbach 2 (DE)**

(72) Erfinder: **Blum, Klaus-Jürgen, Dr., Kleinenbroicher
Strasse 65, D-4050 Mönchengladbach 2 (DE)**

(74) Vertreter: **Bonsmann, Manfred, Dipl.-Ing.,
Kaldenkirchener Strasse 35a,
D-4050 Mönchengladbach 1 (DE)**

(54) **Vorrichtung zur Durchführung einer Akupunktur-Magnettherapie.**

㉗ Die Erfindung befaßt sich mit der physikalischen Therapie von Krankheiten und betrifft eine Vorrichtung zur Akupunktur-Magnettherapie, bei der magnetfelderzeugende Einrichtungen, beispielsweise Permanentmagnete, in einer gegen den zu behandelnden Bereich der Körperoberfläche lose oder fest anzulegenden Einrichtung vorgesehen sind. Die Anordnung der Magnete ist unter Berücksichtigung der topographisch festliegenden Akupunkturpunkte bzw. Meridiane auf ein bestimmtes Krankheitsbild abstimmbar und ermöglicht die Durchführung der Therapie auch durch den medizinischen Laien. Die Ausgestaltung kann so sein, daß die Akupunktur-Magnettherapie mit Akupressur kombiniert wird.

Vorrichtung zur Durchführung einer Akupunktur-Magnettherapie

Die Erfindung betrifft eine Vorrichtung zur Durchführung einer Akupunktur-Magnettherapie, bei der magnetfelderzeugende Einrichtungen in einer gegen den zu behandelnden Bereich der Körperoberfläche anzulegenden Einrichtung vorgesehen sind.

Die heilsame Wirkung von Magnetfeldern ist seit langem bekannt. Die allgemeine positive Wirkung von Magnetfeldern besteht im wesentlichen in einer Optimierung des Stoffwechsels der Zelle, insbesondere des Sauerstoffumsatzes, so daß Nährstoffe schneller antransportiert und verwertet und Abfallstoffe schneller abtransportiert werden können und somit ein krankhafter Prozeß in der Zelle und somit auch in einem betroffenen Gewebe und Körperteil schneller abgebaut und so der Heilungsprozeß beschleunigt herbeigeführt werden kann. Weiterhin ist es bekannt, daß magnetische Felder einen therapeutischen Einfluß auf das Akupunktursystem, die Akupunkturmeridiane und insbesondere die Akupunkturpunkte haben, wobei sowohl im lokalen Bereich um den Akupunkturpunkt herum eine Heilwirkung als auch eine sog. Fernwirkung, die von vielen Punkten ausgehend bekannt ist, erzielt werden kann.

In der physikalischen Medizin ist es bekannt geworden, die heilende Wirkung von Magnetfeldern nutzbar zu machen. Im Rahmen der sog. "Magnetfeld-Therapie" sind als "Magnetotron" bezeichnete Spulen oder Applikatoren bekannt geworden, mit deren Hilfe pulsierende Magnetfelder erzeugt werden, denen bestimmte Körperteile ausgesetzt werden. Die bekannte "Magnetfeld-Therapie" bedingt einen relativ großen apparativen Aufwand, der für die Durchführung einer Magnetfeld-Therapie durch den therapeutischen Laien in Selbstbehandlung zu aufwendig macht.

Weiterhin ist die Anwendung von Permanentmagneten, vorzugsweise Hartferritmagneten, in diesem Zusammenhang bekannt geworden. Diese Magnete werden mittels eines Heftpflasters oder dergleichen - vorzugsweise über Akupunkturpunkten - auf die Haut aufgeklebt, so daß das Magnetfeld den Organismus im Bereich des Akupunkturpunktes bzw. im Bereich der die Akupunkturpunkte verbindenden Kraftlinien, den sog. Meridianen, anregen soll. Hierbei stellt sich jedoch das Problem, das Heftpflaster an der genau richtigen Stelle bei dem jeweiligen Patienten aufzukleben, da der Laie bei der Auffindung der Akupunkturpunkte überfordert ist.

Der Erfindung liegt die Aufgabe zugrunde, eine Vorrichtung der eingangs genannten Art zu schaffen, mittels derer bei geringem Aufwand eine Therapie durch einen Akupunktur-Laien in Selbstbehandlung durchgeführt werden kann, wobei gleichzeitig bestimmte Akupunkturpunkte oder Meridiane unverwechselbar der gewünschten Magnettherapie ausgesetzt werden, so daß die Vorrichtung exakt auf die Behandlung eines Krankheitsbildes abgestimmt werden kann.

Zur Lösung dieser Aufgabe ist erfindungsgemäß vorgesehen, daß die Anordnung der magnetfelderzeugenden Einrichtungen in der genannten Einrichtung der topographischen Akupunkturpunkt- bzw. Meridianlage bzw. deren unmittelbarer Nähe entspricht. Der besondere Vorteil der Erfindung liegt darin, daß die Anordnung der magnetfelderzeugenden Einrichtungen in der Vorrichtung exakt auf ein Krankheitsbild abgestimmt werden kann, wobei die jeweilige, die magnetfelderzeugenden Einrichtungen aufweisende Vorrichtung noch zusätzlich eine stützende, massierende und/oder wärmende Wirkung ausüben kann.

Die magnetfelderzeugenden Einrichtungen können als Permanentmagnete, vorzugsweise Hartferritmagnete ausgebildet sein. Es kann auch vorgesehen sein, daß die magnetfelderzeugenden Einrichtungen als Elektromagnete ausgebildet sind. Die magnetischen Wirkungen können somit sowohl durch von Dauermagneten als auch von Elektromagneten erzeugte statische Magnetfelder erzeugt werden, als auch durch elektromagnetisch erzeugte magnetische Wechselfelder. Um negative Nebenwirkungen mit Sicherheit auszuschließen, werden Magnetfelder verwendet, die am Kopf und Körper eine Feldstärke von 200 G und an Armen und Beinen eine Feldstärke von 2000 G nicht überschreiten.

In weiterer Ausgestaltung der Erfindung kann vorgesehen sein, die Wirkung des Magnetfeldes mit der bekannten Wirkung der Akupressur zu verbinden. Hierzu schlägt die Erfindung vor, die magnetfelderzeugenden Einrichtungen bedingt federnd einzubetten oder zur Erzielung einer zusätzlichen Akupressurwirkung die magnetfelderzeugenden Einrichtungen geringfügig erhaben über die dem zu behandelnden Körperteil zugewandte Seite der Vorrichtung vorstehen zu lassen. In beiden Fällen kann unter Nutzung einer elastischen Wirkung der Vorrichtung (z.B. Bandage) selbst im Zusammenhang mit der Körperbewegung eine milde, aber ständige Akupressurwirkung erzielt werden. In Sonderfällen ist es möglich, die magnetfelderzeugenden Einrichtungen so einzubetten, daß ihre elastische Bewegung in gezielter Richtung erfolgt, d.h., in der Richtung, in der bei der Akupressur der Finger oder beispielsweise ein Akupressurstab bewegt wird.

Eine Ausgestaltung der Erfindung kann darin bestehen, daß die anzulegende Einrichtung als therapeutisches Band oder als therapeutischer Reifen ausgebildet ist.

Wird beispielsweise ein Permanentmagnete aufweisendes Band oder Reifen am Handgelenk getragen und die variable Weite vom Träger so eingestellt wird, daß das Band sich locker um das Handgelenk drehen und in Armlängsrichtung über eine Strecke von ca. 10 bis 15 cm (von der Handwurzel bis zum Ellbogen hin gemessen) gleiten kann, so werden hierdurch eine Reihe von Akupunkturpunkten mit Magnetfeldern behandelt, mit denen therapeutische Effekte zu erzielen sind. Dabei ist zunächst die regulierende Wirkung bei Kreislaufleiden über den Punkt kS 6 zu nennen, der zwei Querfinger proximal der Handgelenksfurche auf der Beugeseite des Unterarmes liegt. Der Punkt kS 7 auf der Mitte der Beugefalte des Handgelenkes hat ebenfalls Kreislaufwirkung. Bei depressiven Krankheitsbildern wirkt der Punkt H 7 am inneren Ende der Beugefalte am kleinfingerseitigen Ende der Beugefalte des Handgelenkes, ebenso der Punkt H 5 etwas proximal davon und der Punkt 3 E 4 in der Mitte der Falte auf der Streckseite des Handgelenkes. Zur Behandlung von Durchblutungsstörungen wird der Punkt Lu 7 am daumenseitigen Ende der Beugefalte des Handgelenkes verwandt, der zwei Querfinger vor dem Handgelenk liegt. Dieser Punkt wird auch verwandt bei der sog. Fitness-Akupunktur. Ein ebenfalls therapeutisch wichtiger Punkt bei Gefäßkrankheiten ist der Punkt Lu 9 am daumenseitigen Ende der Beugefalte des Handgelenkes. Der Punkt Lu 7 wird auch verwandt bei Husten. Ein wichtiger Punkt zur Behandlung von Kopfschmerzen ist wiederum der Punkt 3 e 4 in der Mitte der Falte der Streckseite des Handgelenkes. Für den Fachmann ist ersichtlich, daß auf diese Weise eine Vielzahl weiterer Krankheitsbilder behandelt werden kann.

Das Band kann auch als Stirnband ausgebildet sein, welches sich besonders zur Behandlung von Kopfschmerzen und Migräne-Zuständen eignet. Dabei wird das mit Permanentmagneten versehene Band in Augenbrauenhöhe um den gesamten Kopf fest angebracht. Dies kann zu jeder beliebigen Zeit erfolgen, z.B. während des Schlafes nachts und insbesondere dann, wenn Kopfschmerzen oder Migräne auftreten. Das Stirnband kann auch zur Vorbeugung gegen derartige Beschwerden in den beschwerdefreien Interwallen getragen werden. Hierbei werden besonders wichtige Punkte des Gallenblasenmeridians therapeutisch beeinflußt; ebenso solche mit hormoneller Wirkung wie Punkte des Blasenmeridians sowie Punkte des sog. Lenkergefäßes. Die Anordnung der Magnete kann beispielsweise so sein, daß nachfolgende Akupunkturpunkte beeinflußt werden:

3 E 22
3 E 23 a
Blase 2
Blase 8
Galle 8
Galle 3
LG 16
LG 20
INN TRANG.

Wenn das therapeutische Band bzw. therapeutischer Reifen als Fußgelenksreifen oder Fußgelenksband ausgebildet ist, so können dann, wenn - wie bei dem Handgelenksband - das Band regelmäßig angeordnete Permanentmagnete aufweist und die Weite so eingestellt wird, daß das Band locker um das Fußgelenk gleiten kann und so durch die Bewegung des Gehens über eine Strecke von 10 bis 15 cm im Fußgelenkbereich bewegbar ist, u.a. folgende Krankheitsbilder bzw. Akupunkturpunkte gezielt beeinflußt werden:

Durchblutungsstörungen der Beine - Ni 8 und MP 6
Gallenerkrankungen - G 40
Hormonelle Störungen - MP 6
Erkrankungen des Harntraktes - Ni 3
Ischias - Bl 62, G 40
Krampfaderschmerzen - B 60
Schmerzen allgemein - B 60
Schlafstörungen - B 62 (für Männer)
       - Ni 6 (für Frauen).

Bei der Ausbildung als therapeutisches Band oder Reifen ist die Anwendung der Erfindung nicht auf die genannten Beispiele beschränkt, sondern das Band oder der Reifen können grundsätzlich an allen Körperteilen zur Eigenbehandlung lokaler Beschwerden getragen werden, beispielsweise als Band, welches bei Schmerzen im Bauchbereich um den Körper in Gürtelhöhe oder bei Halsbeschwerden od. dgl. um den Hals getragen wird. Dabei ist dann jeweils die Anordnung der magnetfelderzeugenden Einrichtungen auf die jeweils zu beeinflussenden Akupunkturpunkte oder Meridiane abgestimmt.

In weiterer Ausgestaltung der Erfindung kann vorgesehen sein, daß die anzulegende Einrichtung als elastische, unelastische, halbstarre oder starre Körperstütze, Orthese bzw. Prothese ausgebildet ist.

In weiterer Ausgestaltung der Erfindung kann vorgesehen sein, daß die anzulegende Einreichung als Einlegesohle bzw. Fußbettung oder Schuheinlage ausgebildet ist.

Eine besonders zweckmäßige Ausführungsform der Erfindung liegt in einer Ausbildung der anzulegenden Einrichtung als therapeutische Bandage. Insbesondere bei dieser Ausführungsform tritt durch die stützende, massierende und wärmende Wirkung der Bandage eine kombinierte Wirkung mit den auf ganz bestimmte Körperstellen (Akupunkturpunkte) einwirkenden Magnetfeldern ein. Auch hierbei kann die Anordnung der magnetfelderzeugenden Einrichtungen so getroffen werden, daß eine über die Magnetfeldwirkung hinausgehende Akupressurwirkung erzielt wird.

Im einzelnen kann vorgesehen sein, daß die Bandage einschichtig oder mehrschichtig ausgebildet ist. Im letzteren Fall können zwischen einer vorzugsweise elastischen Außenschicht und einer hautfreundlichen Innenschicht die magnetfelderzeugenden Einrichtungen, vorzugsweise Permanentmagnete, angeordnet sein. Mit dieser Maßnahme kann eine gute Luftdurchlässigkeit erzielt werden; d.h., ein unerwünschter Feuchtigkeitsstau wird vermieden.

In weiterer Ausgestaltung weist die Bandage an der dem zu behandelnden Körperteil zugewandten Seite nach Maßgabe der Akupunkturpunkte und/oder Meridiane verlaufende, abgesteppte Aufnahmetaschen für die Aufnahme und lokale Festlegung der Permanentmagnete auf. Die Erfindung geht dabei von der Überlegung aus, daß die topographische Anordnung von Akupunkturpunkten und Meridianen grundsätzlich bekannt ist, so daß der behandelnde Arzt die Anordnung der Magnete so vornehmen kann, daß die optimale Heilwirkung erzielt wird. Eine in dieser Weise vorbereitete Bandage kann dann vom Patienten ohne ärztliche Hilfe angelegt werden.

Das Abstellen einer solchen Bandage auf eine bestimmte Therapie kann beispielsweise dadurch erleichtert

- 8 -                    0100050

werden, daß die Permanentmagnete lösbar (z.B. durch
Kleben) in Form von Gewebestreifen od. dgl. auf der
dem zu behandelnden Körperteil zugewandten Seite der
Bandage befestigt sind, oder es können in weiterer
Ausgestaltung der Erfindung Aufnahmetaschen, Streifen
od. dgl. rasterartig über die dem zu behandelnden Körperteil zugewandte Seite der Bandage verlaufend angeordnet sein, so daß in jedem Fall eine individuelle
Anpassung der Permanentmagnete mit Bezug auf die Bandage einerseits und die Akupunkturpunkte andererseits
möglich ist.

Es ist aber auch nach einem weiteren eigenständigen
Merkmal der Erfindung vorteilhaft, beispielsweise eine
Bandage auf ein bestimmtes zu therapierendes Krankheitsbild abzustimmen, d.h., die Möglichkeit zur Anordnung
von Magneten auf ein Minimum zu reduzieren, so daß
eine bestimmte Bandage nur für eine bestimmte Therapie benutzt werden kann; dies ermöglicht dem Laien,
ohne weitere ärztliche Hilfe auszukommen, wenn ihm
lediglich Bandage und Magnete zur Verfügung gestellt
werden.

Im einzelnen können bei der Ausführung der Erfindung
Permanentmagnete unterschiedlicher Feldstärke - auch
mit Bezug auf eine einzige Bandage - zur Anwendung kommen; hierdurch ist es möglich, die unterschiedliche
"Ansprechcharakteristik" bestimmter Akupunkturpunkte
zu berücksichtigen. Wie bereits erwähnt, können die
magnetfelderzeugenden Einrichtungen auch so ausgebildet sein, daß magnetische Wechselfelder abgegeben werden.

Die Erfindung kann bei einer Vielzahl unterschiedlicher Bandagen Anwendung finden; bevorzugt werden beispielsweise Kniebandagen, Leib- bzw. Lendenbandagen,
Ellenbogenbandagen, Fußgelenk- und Fußbandagen bzw.

Nacken- und Schulterbandagen in der erfindungsgemäßen Weise ausgebildet.

Die Erfindung wird nachfolgend anhand der in den Zeichnungen dargestellten Ausführungsbeispiele näher erläutert. Es zeigen:

Fig. 1    die Vorderansicht einer Kniebandage in perspektivischer Darstellung;

Fig. 2    die Rückansicht der Kniebandage nach Fig. 1;

Fig. 3 bis 10   schematisch die Vorder- bzw. Rückansicht verschiedener Bandagen mit Bezug auf den menschlichen Körper;

Fig. 11 eine Draufsicht auf eine erfindungsgemäß gestaltete Einlegesohle;

Fig. 12 einen Schnitt längs der Linie XII-XII in Fig. 11.

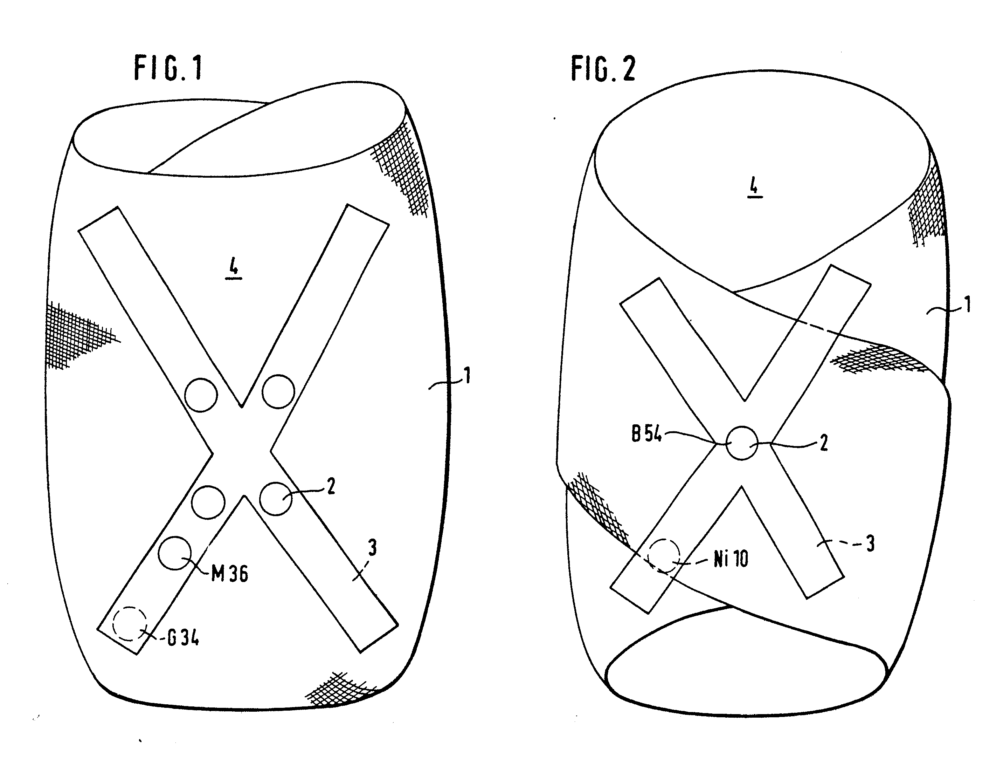

Die Erfindung wird ausführlich anhand der Fig. 1 und Fig. 2 erläutert. Die Fig. 1 und Fig. 2 zeigen eine als Kniebandage 4 ausgebildete Bandage 1, die gemäß der Vorderansicht nach Fig. 1 vorne geschlossen und im Bereich der Kniekehle nach Maßgabe zweier sich kreuzender Bänder ausgebildet ist. Die Kniebandage 4 besteht in bekannter Weise aus einem elastischen Gewebe. Aus der Fig. 1 ist ersichtlich, daß an der der Kniescheibe zugewandten Seite zwei streifenförmige Aufnahmetaschen 3 kreuzweise angeordnet sind. Diese kreuzweise Anordnung ist bei der Kniebandage 4 deswegen gewählt worden, weil sie auf die Behandlung von Knieschmerzen abgestellt ist. Dementsprechend ist in einer der Aufnahmetaschen der mit der Bezeichnung M 36 versehene Permanentmagnet angeordnet, der bei Benutzung

BAD ORIGINAL

der Bandage über dem Akupunkturpunkt "tsu-san-li" liegt, welcher sich unter der Ringfingerspitze befindet, wenn man die Handinnenfläche gerade auf die Kniescheibe legt. Begleitend werden weitere mit 2 bezeichnete Permanentmagnete über den sogenannten lokalen Punkten angeordnet. Wichtig ist bei der an dieser Stelle beschriebenen Therapie, daß der mit M 36 bezeichnete Punkt mit einem weiteren mit B 54 bezeichneten Punkt in der Mitte der Kniekehle kombiniert wird. Dieser Punkt wird in der Akupunktur mit "wei-chung" bezeichnet. Die den Punkten M 36, B 54 sowie den lokalen Punkten zugeordneten Magnete sind in vollen Linien in die Fig. 1 und 2 eingezeichnet worden, um deutlich zu machen, daß gemäß der Therapie der Behandlung von Knieschmerzen diese Punkte bzw. Magnete in einem Wirkzusammenhang stehen.

Es wird darauf hingewiesen, daß bei anderer Therapie, Magnetfeldwirkungen auf den Punkt M 36 (tsu-san-li) mit an anderen Körperstellen angeordneten Bandagen und zugeordneten Magneten in völlig geänderter Weise kombiniert werden können; der Vollständigkeit halber sei darauf hingewiesen, daß der Akupunkturpunkt tsu-san-li der wesentliche psychische Entspannungspunkt ist, d.h., er dient unter anderem der Behandlung von

der psychischen Entspannung bei Herzklopfen, Nasenbluten, Asthma, Phantomschmerz, Prostataentzündung, Stottern, Suchtkrankheiten, Verstopfung sowie

der Funktionsanregung im Bereich der Sexualsphäre sowie

der Behandlung von Schlafstörungen, Nervosität und Reizbarkeit, Magenschmerzen, Krampfaderschmerzen, Bettnässen, Depressionen sowie ebenfalls

der Förderung der allgemeinen körperlichen Fitness.

Es ist ersichtlich, daß die Anordnung der Magnete mit Bezug auf eine bestimmte Bandage aus den vorgenannten Gründen von einem fachkundigen Arzt vorgenommen werden sollte.

In Fig. 1 ist weiter gestrichelt ein Permanentmagnet angedeutet, der über dem mit G 34 bezeichneten Akupunkturpunkt liegt. Bei diesem Akupunkturpunkt handelt es sich um den Punkt "dang-nang-dian", der sich drei Querfinger unterhalb des tastbaren Wadenbeinköpfchens befindet, allgemein als "Gallenpunkt" bezeichnet wird und unter anderem der Therapie von Verspannungen im Leber/Gallesystem, wie Gallenkoliken, aber auch der Leberfunktionsanregung sowie Behandlung von Kopfschmerzen, Schlafstörungen und Völlegefühl dient. Die gestrichelte Anordnung des Permanentmagneten über dem Punkt G 34 soll darauf hinweisen, daß in dem gezeigten Ausführungsbeispiel (Therapie von Knieschmerzen) der Permanentmagnet natürlich nicht eingesetzt würde, wenngleich er im Bereich der kreuzweise angeordneten Aufnahmetaschen bei einer Gallentherapie Anwendung finden müßte.

Ähnliches gilt für den mit Ni 10 bezeichneten Akupunkturpunkt gemäß Fig. 2. Der Akupunkturpunkt Ni 10 ist der Punkt "yin-ku", der sich (bei gebeugtem Knie) am inneren Ende der Kniegelenkfalte befindet. Er ist beispielsweise bei Nierenstörungen zu beachten.

Mit dem vorgenannten Anwendungsbeispiel (Therapie von Knieschmerzen) wird deutlich, daß die Anordnung der Permanentmagnete auf ein bestimmtes Krankheitsbild abgestimmt sein muß; es wird aber auch verdeutlicht, daß eine Bandage möglicherweise für mehrere Therapien geeignet ist, wenn entsprechende Aufnahmemöglichkei-

0100050

ten für eine ausreichend verschiebungssichere Anordnung von Permanentmagneten vorgesehen sind.

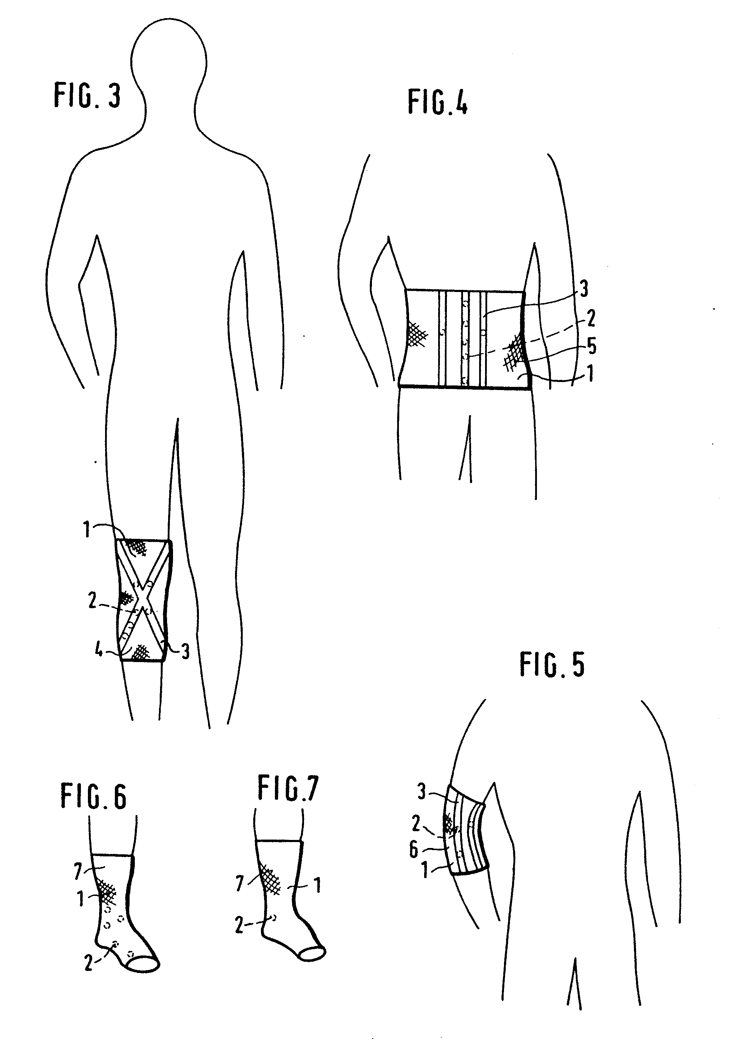

In den Fig. 3 bis 10 sind weitere Ausführungsformen von Bandagen dargestellt. Die bei den einzelnen Bandagen dargestellten Aufnahmemöglichkeiten sind - ohne daß hier auf die einzelnen Akupunkturpunkte, deren Bezeichnung und deren Wirkung detailliert eingegangen werden soll - geeignet im Rahmen der nachfolgend aufgeführten Indikationen:

Fig. 3 (Kniebandage 4; Vorderansicht)
Indikationen:

        Knieschmerzen
        Kniearthrose
        Meniskusbeschwerden
        Kniescheibenschmerzen
        zur Beruhigung und Ausgeglichenheit
        bei Magenbeschwerden

Fig. 4 (Leib- bzw. Lendenbandage 5; Vorderansicht)
Indikationen:

        Kopfschmerz und Migräne (bei Wetterfühligkeit)
        Magenschmerzen
        Schwindel
        Anregung der Sexualsphäre
        Blasenstörungen

Fig. 5 (Ellenbogenbandage 6; Seiten-, Vorderansicht)
Indikationen:

        Ellenbogenschmerz
        Tennisellenbogen
        Verstopfung und Blähungen

BAD ORIGINAL

Durchblutungsstörungen der Hände und Arme Handallergie

Ellenbogen-Arthrose

Fig. 6 (Fußgelenk- und Fußbandage 7; Vorder-, Innenansicht)

Indikationen:

Appetitlosigkeit

Aufstoßen

Bettnässen

Blasenstörungen

Durchblutungsstörungen der Beine Hautjucken

Kopfschmerzen, Migräne (hormonell)

Fig. 7 (Fußgelenk- und Fußbandage 7; Außenansicht) Indikationen:

Ischias

Krampfaderschmerzen

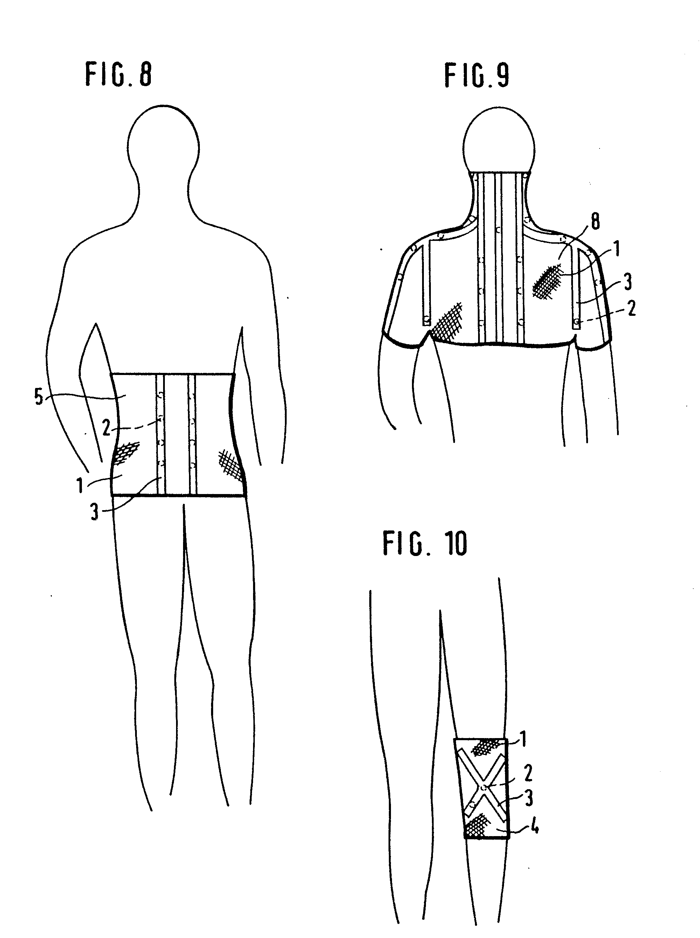

Fig. 8 (Leib- bzw. Lendenbandage 5; Rückansicht) Indikationen:

Ischias

Rückenschmerzen

Kreuzschmerzen

Bandscheibe

Verschleiß der Lendenwirbelsäule

Fig. 9 (Nacken- und Schulterbandage 8; Rückansicht) Indikationen:

Nackenschmerz

Halswirbel- und Bandscheibenschmerzen

Kopfschmerz mit Nackenschmerz

0100050

Migräne
Schwindel
Schulterarmschmerz
Schultergelenk-Arthrose

Fig. 10 (Kniebandage 4; Rückansicht)
Indikationen:

Nierenfunktionsanregung
Blasenstörung.

Die Erfindung bleibt nicht auf die vorstehend beispielsweise beschriebenen Bandagen beschränkt; hier sind nicht nur weitere Bandagen an sich, sondern insbesondere weitere Ausgestaltungsformen der Aufnahmemöglichkeiten für die Permanentmagnete mit Bezug auf die einzelnen Bandagen in Abstimmung auf die jeweils anzuwendende Therapie denkbar. Dies gilt auch insbesondere für die Kombination mehrerer Bandagen untereinander.

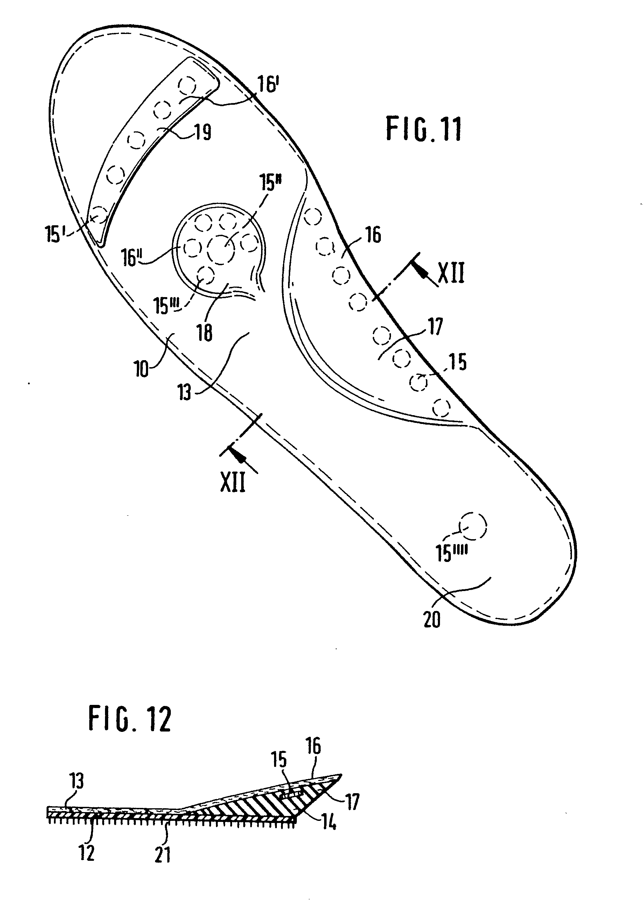

Die in Fig. 11 dargestellte medizinisch therapeutische Einlegesohle ist insgesamt mit 10 bezeichnet.

Wie aus dem Querschnitt gemäß Fig. 12 ersichtlich, besteht die Einlegesohle aus einer Einlage 12, einem dieser Einlage oberseitig anliegenden Massagegewebe 13 sowie im Rahmen örtlicher Erhöhungen angeordneten Zwischenlagen 14 aus einem elastischen Material, welches hier als Weichgummi ausgebildet ist. In vorbestimmtem Abstand unterhalb des Massagegewebes 13 sind, wie aus Fig. 12 ersichtlich, Hartferritmagnete 15 eingebettet. Die Einbettung der Hartferritmagnete 15, wie sie im Schnitt durch das Längsgewölbe 17 in Fig. 12 dargestellt ist, ist in ähnlicher Weise auch im Bereich der Pelotte 18 und des Zehenwulstes 19 getroffen.

BAD ORIGINAL

Wie aus Fig. 11 weiter erkennbar, ist die Anordnung der Permanentmagnete 15 im Längsgewölbe 17 linear ausgebildet, so daß sich hier eine Erhöhung 16 in Form eines elastischen Steges 17 ergibt. Eine weitere, mit 16' bezeichnete wesentliche Erhöhung ergibt sich durch die Anordnung der Permanentmagnete 15' im Bereich des Zehenwulstes 19. Fig. 11 läßt erkennen, daß der Zehenwulst 19 etwa kurvenförmig verlaufend ausgebildet ist, wobei er sich in seiner Breite zur Sohlenaußenseite hin verringert.

Schließlich wird eine dritte besonders markante, mit 16'' bezeichnete Erhöhung im Bereich Pelotte 18 verwirklicht; die Anordnung der Magnete ist hier so getroffen, daß sich um einen zentrischen Permanentmagneten 15'' etwa halbkreisförmig mehrere kleinere Magnete 15''' gruppieren, derart, daß der Halbkreis zum Fersenteil 20 hin offen ausgebildet ist. Im Bereich des Fersenteiles 20 ist ein zusätzlicher Permanentmagnet 15'''' zwischen Einlage 12 und Massagegewebe 13 eingebettet.

Mit dieser Anordnung kann u.a. auf folgende Akupunkturpunkte eingewirkt werden:

MP 2, 3, 4 und 5
Ni 1 und 2
Le 2 und 3
M 45, 42 und 41
G 40, 41 und 43
Wl 62, 64, 65 und 67.

Zur rutschsicheren Befestigung im zugeordneten Schuhwerk ist unterhalb der Einlage 12 eine rutschfeste, mit 21 bezeichnete Bodensohle angeordnet.

Patentansprüche

1. Vorrichtung zur Durchführung einer Akupunktur-Magnettherapie, bei der magnetfelderzeugende Einrichtungen in einer gegen den zu behandelnden Bereich der Körperoberfläche anzulegenden Einrichtung vorgesehen sind, dadurch gekennzeichnet, daß die Anordnung der magnetfelderzeugenden Einrichtungen (2,15,15',15'',15''',15'''') in der genannten Einrichtung (1, 10) der topographischen Akupunkturpunkt- bzw. Meridianlage bzw. deren unmittelbarer Nähe entspricht.

2. Vorrichtung nach Anspruch 1, dadurch gekennzeichnet, daß die magnetfelderzeugenden Einrichtungen als Permanentmagnete, vorzugsweise Hartferritmagnete, ausgebildet sind.

3. Vorrichtung nach Anspruch 1, dadurch gekennzeichnet, daß die magnetfelderzeugenden Einrichtungen als Elektromagnete ausgebildet sind.

4. Vorrichtung nach Anspruch 3, dadurch gekennzeichnet, daß die magnetfelderzeugenden Einrichtungen zur Erzeugung von magnetischen Wechselfeldern ausgebildet sind.

5. Vorrichtung nach einem der Ansprüche 1 bis 4, dadurch gekennzeichnet, daß zur Erzielung einer zusätzlichen Akupressurwirkung die magnetfelderzeugenden Einrichtungen (2,15,15',15'',15''', 15'''') bedingt federnd elastisch in die Einrichtung (1, 10) eingebettet sind.

6. Vorrichtung nach einem der Ansprüche 1 bis 5, dadurch gekennzeichnet, daß die magnetfelderzeugenden Einrichtungen zur Erzielung einer zusätzlichen Akupressurwirkung geringfügig erhaben über die dem zu behandelnden Körperteil zugewandte Seite der Einrichtung vorstehend angeordnet sind.

7. Vorrichtung nach einem der Ansprüche 1 bis 6, dadurch gekennzeichnet, daß die anzulegende Einrichtung als therapeutisches Band ausgebildet ist.

8. Vorrichtung nach einem der Ansprüche 1 bis 6, dadurch gekennzeichnet, daß die anzulegende Einrichtung als therapeutischer Reifen ausgebildet ist.

9. Vorrichtung nach einem der Ansprüche 1 bis 6, dadurch gekennzeichnet, daß die anzulegende Einrichtung als elastische, unelastische, halbstarre oder starre Körperstütze, Orthese bzw. Prothese ausgebildet ist.

10. Vorrichtung nach einem der Ansprüche 1 bis 6, dadurch gekennzeichnet, daß die anzulegende Einrichtung als Einlegesohle (10) bzw. Fußbettung oder Schuheinlage ausgebildet ist.

11. Vorrichtung nach einem der Ansprüche 1 bis 6, dadurch gekennzeichnet, daß die anzulegende Einrichtung als therapeutische Bandage (1) ausgebildet ist.

12. Vorrichtung nach Anspruch 11, dadurch gekennzeichnet, daß die Bandage (1) einschichtig oder mehrschichtig ausgebildet ist, und Permanentmagnete (2) zwischen einer vorzugsweise elastischen Aussenschicht und einer hautfreundlichen Innenschicht angeordnet sind.

13. Vorrichtung nach Anspruch 11 oder 12, dadurch gekennzeichnet, daß die Bandage (1) an der dem zu behandelnden Körperteil zugewandten Seite nach Maßgabe der Akupunkturpunkte und/oder Meridiane verlaufende abgesteppte Aufnahmetaschen (3) für die Aufnahme und lokale Festlegung der Permanentmagnete aufweist.

14. Vorrichtung nach Anspruch 13, dadurch gekennzeichnet, daß die Aufnahmetaschen (3) streifen- oder rasterartig über dem zu behandelnden Körperteil verlaufend angeordnet sind.

15. Vorrichtung nach einem der Ansprüche 13 und 14, dadurch gekennzeichnet, daß die Anordnung der Aufnahmetaschen (3) mit Bezug auf die Bandage (1) auf ein bestimmtes zu therapierendes Krankheitsbild abgestimmt ist.

16. Vorrichtung nach Anspruch 12, dadurch gekennzeichnet, daß die Permanentmagnete (2) lösbar (z.B. durch Kleben) in Form von Gewebestreifen od. dgl. auf der Bandage befestigt sind.

17. Vorrichtung nach einem der Ansprüche 9 bis 14, gekennzeichnet durch die Verwendung von Permanentmagneten (2) unterschiedlicher Feldstärke mit Bezug auf eine einzige Bandage (1).

18. Vorrichtung nach einem der Ansprüche 11 bis 17, dadurch gekennzeichnet, daß die Bandage (1) als Kniebandage (4), Leib- bzw. Lendenbandage (5), Ellenbogenbandage (6), Fußgelenk- und Fußbandage (7), Nacken- und Schulterbandage (8), Stirnbandage, Halswirbelsäulenbandage, Fußknöchelbandage, Handgelenkbandage, Bandage an Hand und Finger
oder als Fingerring ausgebildet ist.

FIG.1

FIG.2

B54

Ni 10

M36

G34

1  2  3  4

FIG. 3

FIG.4

FIG.5

FIG.6

FIG.7

FIG. 8

FIG. 9

FIG. 10

FIG. 11

FIG. 12